Europäisches Patentamt

European Patent Office

Office européen des brevets

(11) Publication number: **0 261 671 B1**

# EUROPEAN PATENT SPECIFICATION

(45) Date of publication of patent specification: **13.04.94**    (51) Int. Cl.5: **A61K 47/48**, C07K 15/00

(21) Application number: **87113929.1**

(22) Date of filing: **23.09.87**

Divisional application 93113917.4 filed on 23/09/87.

(54) **Recombinant pseudomonas exotoxin: construction of an active immunotoxin with low side effects.**

(30) Priority: **24.09.86 US 911227**

(43) Date of publication of application:
**30.03.88 Bulletin 88/13**

(45) Publication of the grant of the patent:
**13.04.94 Bulletin 94/15**

(84) Designated Contracting States:
**AT BE CH DE ES FR GB GR IT LI LU NL SE**

(56) References cited:
**WO-A-83/03971**
**WO-A-86/00090**
**US-A- 4 470 924**

**CHEMICAL ABSTRACTS, vol. 101, no. 17, 22. October 1984, p. 166, abstract no. 145086w Columbus, Ohio, US; M.A. MOZOLA et al.: "Cloning and expression of a gene segment encoding the enzymatic moiety of Pseudomonas aeruginosa exotoxin A", & J. BACTERIOL. 1984, 159(2), 683-7**

(73) Proprietor: **Pastan, Ira**
**11710 Beall Mountain Road**
**Potamac Maryland 20854(US)**

Proprietor: **Fitzgerald, David J.**
**1731 Ladd Street**
**Silver Spring Maryland 20902(US)**

Proprietor: **Adhya, Sankar**
**1440 Kings Grant Street**
**Gaithersbourg Maryland 20878(US)**

(72) Inventor: **Pastan, Ira**
**11710 Beall Mountain Road**
**Potamac Maryland 20854(US)**
Inventor: **Fitzgerald, David J.**
**1731 Ladd Street**
**Silver Spring Maryland 20902(US)**
Inventor: **Adhya, Sankar**
**1440 Kings Grant Street**
**Gaithersbourg Maryland 20878(US)**

(74) Representative: **Patentanwälte Grünecker,**
**Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 261 671 B1

Rank Xerox (UK) Business Services
(3.10/3.09/3.3.3)

PROC. NATL. ACAD. SCI. USA, VOL. 81, May 1984, pp. 2645-2649, Biochemistry, US; G.L. GRAY et al.: "Cloning nucleotide sequence, and expres- sion in Escherichia coli of the exotoxin A structural gene of Pseudomonas aerugionosa", p. 2647, p. 2648

INFECTION AND IMMUNITY, vol. 52, no. 3, June 1986, pp. 756-762, American Soc. for Microbiology, US; J.K.S. CHIA et al.: "Func- tionally distinct monoclonal antibodies reac- tive with enzymatically active and binding domains of Pseudomonas aeruginosa Toxin A", p. 757

CHEMICAL ABSTRACTS, vol. 103, no. 17, 28. Oct. 1985, p. 221, abstract no. 135371z, Co- lumbus, Ohio, US; R.C. CLOWES et al.: "Clon- ing of an enzymically active segment of the exotoxin-A gene of Pseudomonas aeruginosa", & BASIC LIFE SCI, 1985, 30 (Plasmids Bact.) 777-90

PROC. NATL. ACAD. SCI, USA, vol. 83, Sept. 1986, pp. 6627-6630, US; D.J. FITZGERALD et al.: "Antitumor effects of an immunotoxin made with Pseudomonas exotoxin in a nude mouse model of human ovarian cancer"

PROC. NATL. ACAD. SCI, USA, vol. 84, July 1987, pp. 4538-4542, US; V.K. CHAUDHARY et al.: "Activity of a recombinant fusion protein between transforming growth factor type al- pha and Pseudomonas toxin

PROC. NATL. ACAD. SCI. USA, vol. 83, March 1986, pp. 1320-1324 US; V.S. ALLURED et al.: "Structure of exotoxin A of Pseudomonas aeruginosa at 3.0-Angstrom resolution"

BIOLOGICAL ABSTRACTS, abstract no. 32106293 US; S.C. JOHNSON et al.: "Genetic construction and characterization of an ex- otoxin A structural gene derivative Tox-A' from Pseudomonasaeroginosa", & AB- STRACTS OF THE ANNUAL MEETING OF THE AMERICAN SOCIETY FOR MICROBIOLOGY (US), 1987, vol. 87, no 9, p. 33

## Description

Background of the Invention

Toxins are extremely potent cell-killing agents that are responsible for many human diseases. Because of their high activity, these agents have been attached to monoclonal antibodies in order to form cytotoxic agents (immunotoxins) which specifically bind to target cells. These immunotoxins are, therefore, most useful in cancer therapy.

Psuedomonas exotoxin A (PE) is an extremely active monomeric protein (molecular weight 66Kd), secreted by Pseudomonas aeruginosa, which inhibits protein synthesis in eukaryotic cells through the inactivation of elongation factor 2 (EF-2) by catalyzing its ADP-ribosylation (catalyzing the transfer of the ADP ribosyl moiety of oxidized NAD onto EF-2).

The intoxication process is believed to proceed by the following steps: First, PE binds to cells through a specific receptor on the cell surface. Next, the PE-receptor complex is internalized into the cell. Finally, PE is transferred to the cytosol where it enzymatically inhibits protein synthesis. The transfer process is believed to occur from an acidic compartment, since cellular intoxication is prevented by weak bases such as $NH_4^+$, which raises the pH in acidic vesicles. Upon exposure to acidic conditions, the hydrophobic domain of PE enters into the membrane, resulting in the formation of a channel through which the enzymatic domain, in extended form, passes into the cytosol.

WO 83/03 971 discloses a prophetic scheme for producing a hybrid protein which comprises the enzymatically active Fragment A of diphtheria toxin and a portion of the binding domain of a cell-specific polypeptide ligand, said portion of said binding domain theoretically having the ability to cause said hybrid protein to bind selectively to a predetermined class of cells to be attacked by said enzymatically active Fragment A.

U.S. patent 4,545,985 teaches that pseudomonas toxin can be chemically coupled to an antibody or growth factor. However, these chemically linked toxins have been shown to have undesirable levels of toxicity. It would be useful, therefore, to have toxins highly targeted for specific cell types without the generalized cell killing normally associated with these toxins.

PE-containing immunotoxins are constructed by first reacting native PE with iminothiolane. This reaction serves both to introduce two new sulfhydryl groups used for coupling an antibody to the toxin, and to inactivate the binding of PE to its own receptor. This approach relies on the chemical inactivation of PE-binding sites in order to minimize undesirable side effects due to the binding of PE to cells with PE receptors. While this approach has been reasonably successful in producing a specific cell killing reagent in tissue culture and in tumor-bearing mice, it has not been possible to administer more than 2 ug of PE immunotoxin to a 20 gram mouse or 1 mg to a 3 kilogram monkey or 4 mg to an adult human, due to the toxic side effects of the immunotoxin. It is therefore desirable to be able to administer larger amounts of immunotoxins to achieve greater killing of tumor cells. The present invention fulfills this desire by providing an immunotoxin with high potency and low toxicity. Furthermore, to overcome the above-noted reliance on chemical inactivation of the PE binding sites, the present invention incorporates recombinant DNA techniques to clone the complete toxin gene (or segments of it) in order to express at high levels the full length toxin molecule (or portions of it) containing different functional domains, including one which lacks the cell binding domain. See example 1 for a comparative examination of these clones.

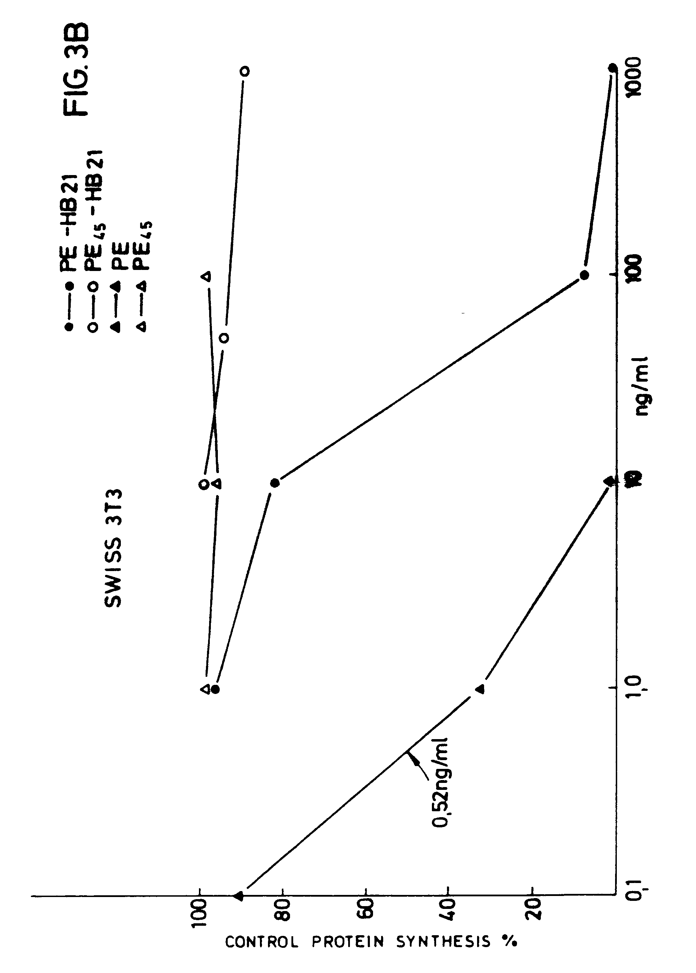

The three-dimensional structure of PE has been determined by x-ray crystallography. As shown in Figure 2, the PE molecule contains three structurally distinct domains: Domain I contains amino acid residues 1 to 252 (Domain Ia), and 365 to 404 (Domain Ib); domain II contains amino acid residues 253 to 364; and domain III contains amino acid residues 405 to 613.

Plasmids have been constructed which express various portions of the PE molecule, providing the ability to correlate different structural domains with various functional activities and to determine (1) which portions of the molecule are responsible for cell recognition (binding , structural domain I, amino acid 1-252); (2) which portion is required for enzymatic activity (ADP ribosylating activity, domain III plus a portion of domain Ib, amino acid 385-613); (3) which portion is responsible for translocation across cell membrane (domain II). The evidence that structural domain Ia is involved in cell recognition is that proteins produced by plasmids without domain Ia but containing domain II, Ib and III, are not cytotoxic by themselves and do not show competitive inhibition of the cytotoxicity of intact toxins, whereas a plasmid encoding domain Ia, but missing other domains, blocked PE cytotoxicity of sensitive cells. PE from plasmids with a deletion of the first half of structural domain II exhibit both PE blocking activity and ADP-ribosylation activity, but these molecules lost all cell killing activity. Plasmids which encode only structural domain III produce large amounts of the protein, but lack detectable enzymatic activity (ADP-ribosylation). However, plasmids which

3

encode all of structural domain III plus adjacent amino acids from structural domain Ib express large amounts of the protein and their ADP-ribosylation activity is high.

Based on the three dimensional structure of PE, plasmids have been constructed which express different portions of PE. The protein pattern of the cells expressing the different constructions was analyzed by SDS gel electrophoresis and the ADP ribosylating activity of the recombinant toxins was measured. Of these plasmids (described in Example 1) plasmid pJH8, containing amino acids 253 to 613 (Domains Ib, II, and III), is able to encode large amounts of modified PE exhibiting low toxicity to human cells, but remaining enzymatically very active.

Taken together, the plasmid patterns indicate that structural domain Ia is a receptor binding domain, that the first half of structural domain II is required for translocation of the toxin from a host cell's endocytic vesicle to the cytosol, and that structural domain III alone is not sufficient to express full ADP-ribosylation activity.

When administered to animals, PE characteristically produces death due to liver failure. Immunotoxins made with PE also attack the liver and, when given in large amounts, produce death due to liver toxicity. The experiments shown in Table III indicate that Domain Ia is responsible for cell binding and indicate that a PE molecule in which Domain Ia is deleted is less toxic to mice than native PE. The data shown in Example 4 support this conclusion, indicating that modified PE is at least 200-fold less toxic to mice than native PE. Example 5 shows that when the protein made by pJH8 is purified and coupled to an antibody to the human transferrin receptor, an immunotoxin is created that is approximately as active as an immunotoxin containing native PE but is 100-fold less toxic on nontarget cells (mouse cells).

One aspect of the present invention, therefore, is that PE molecules with a deletion of Domain Ia are effective immunotoxins with diminished side effects including diminished liver toxicity.

Description of the Figures

Figure 1 shows the construction of the plasmids of the present invention used for the expression of different domains of PE.

Figure 2 in a simplified map of the different sized PE molecules produced as a part of the present invention.

Figure 3 shows the effect of $PE_{45}$-HB21 and PE-HB21 on protein synthesis in human KB cells. $PE_{45}$ is the toxin protein lacking Domain I encoded by plasmid pJH8. Cells were incubated for 24 hours with the appropriate immunotoxin and then incubated with $^3$H-leucine for 1 hour. Incorporation of ($^3$H)-leucine into protein was measured. In panel 3B Swiss 3T3 cells were incubated for 24 hours with either native pseudomonas toxin (PE), native pseudomonas toxin coupled to HB21, $PE_{45}$ alone, or $PE_{45}$ coupled to HB21. Cells were exposed to these agents for 24 hours and then protein synthesis measured for 1 hour as above.

Specific Disclosure of the Invention

The present invention provides a modified Pseudomonas exotoxin which comprises ADP ribosylating activity and the ability to translocate across a cell membrane and wherein the exotoxin comprises a modification in the receptor binding domain Ia of the native toxin sufficient to render the modified toxin less toxic to human or animal cells in vitro and less toxic to the liver when administered in vitro relative to an unmodified Pseudomonas exotoxin. The modified toxin and the immunotoxin made from it have markedly diminished non-specific toxicity on human and mouse cells in tissue culture and with greatly diminished toxicity in mice in vivo.

Genomic DNA is obtained by completely digesting a strain of Pseudomonas aeruginosa with EcoRI and PstI. Although any strain of P. aeruginoia is suitable for use in this invention, the preferred strain is PA103, a strain which produces a large amount of active toxin. DNA fragments ranging in size from 2.6 to 2.9 kb are isolated from the genomic DNA library, and ligated with a 2.7 kb DNA fragment from plasmid pUC13 cut with EcoRI and PstI. Plasmid pUC13 is commercially available from Boehringer Mannheim. A suitable host, preferably an E. coli strain, is then transformed with the ligated products (the preferred strain of E. coli is HB101, commercially available from Bethesda Research Laboratories). The 2.7 kb DNA fragment derived from ptoxETA, a plasmid which contains the PE structural gene, is used as a probe and plasmid DNA from several positive colonies were prepared and characterized by Southern blotting. The different sizes of recombinant PE are then expressed in a suitable host. A host which carries the bacteriophage T7 RNA polymerase gene in lysogenic and inducible form [BL21(DE$_3$)] is preferred, and is obtainable from Dr. F.W. Studier at Brookhaven National Laboratories. Also preferred are plasmids which carry the bacteriophage late

4

T7 promoter and AMP$^r$ and Shine-Dalgarno box--pAR2156, also obtainable from Dr. Studier.

As is shown in the Examples, the preferred plasmid is pJH8. pJH8 contains a DNA fragment of 5.1 kb and is capable of expressing Domains II, Ib, and III of PE. This plasmid is produced by partially cutting plasmid pJH4 with AvaI. The linearized DNA fragment is then completely cut with HindIII in order to obtain a 5.1 kb DNA fragment which has AvaI and HindIII sites at its ends. This fragment is then incubated with S1 nuclease to remove its cohesive ends, followed by ligation with T4 ligase to form plasmid pJH8.

Expression of Recombinant Toxins in BL21(DE$_3$).

BL21(DE$_3$) containing plasmids for expression of different sizes of PE is cultured in LB broth with 50 ug Ampicillin/ml at 37°C. When absorbance at 650 nm reaches 0.3, IPTG (isopropyl beta-D-thio-galactopyranoside) is added to the culture at a final concentration of 1mM. Cells are harvested 90 minutes later and analyzed for the amount of recombinant toxins by SDS-PAGE, immunoblotting, ADP-ribosylation, and cell killing experiments.

SDS-PAGE and Immunoblotting

Cells pellets are dissolved in Laemmli buffer. Samples are boiled for 5 minutes prior to application to a 0.1% SDS, 10% acrylamide slab gel. For immunoblotting, samples after electrophoresis are transferred to a nitrocellulose paper, followed by reaction with antibody to PE, then a second antibody (goat anti-rabbit) and staining. The antibody to PE is obtained by hyperimmunizing rabbits with glutaraldehyde (0.2%) reacted PE (250 ug of PE per injection). An IgG fraction was prepared for immunoblotting.

Assay of ADP-ribosylation Activity

Known procedures are used for assay of ADP-ribosylation activity. Briefly, rabbit reticulocyte preparations or wheat germ extracts enriched with elongation factor 2 (EF-2) are used as a source of EF-2. Assays (500 ul total volume) contain about 10 pmole of EF-2, 37 pmole of $^{14}$C-NAD (0.06 u Ci), 0.25 to 1.25 ug of PE and buffer (40 mM DTT, 1 mM EDTA, and 50 mM Tris, pH 8.1). Activity is measured as pmoles of NAD transferred to EF-2 in 30 minutes. A standard curve of known concentrations of PE is established and used to determine the activity of PE in extracts from E. coli. After incubation for 30 minutes at 37°C, 0.5 ml 12% TCA is added to each assay mixture. The assay mixtures are then set in an ice bath for 15 minutes, followed by centrifugation at 4°C, 3,000 xg for 10 minutes. The pellet is washed with 1 ml 6% TCA and centrifuged as above. The pellet is then measured for $^{14}$C radioactivity in a liquid scintillation counter as the index of the ADP-ribosylation activity.

Cell Cytotoxicity Test

Tests of the cytotoxic activity of the modified PE are performed in NIH 3T3 cell cultures and human KB cells. NIH 3T3 cells or KB cells are seeded 24 hours prior to the cytotoxicity test in a 24-well tissue culture plate at a density of 2 x 10$^4$ cells per well. After incubation for 48 hours with various concentrations of PE or protein extracts isolated from BL21(DE$_3$) with plasmids which express different sizes of PE, the monolayers are stained with methylene blue to detect the surviving cells. The results are shown in Table 1.

Inhibition of Protein Synthesis

Assays for the inhibition of protein synthesis by PE or PE with extracts from BL21(DE$_3$)/pJH8 are performed in Swiss 3T3 cell cultures. Swiss 3T3 cells are seeded one day before assay in 24-well tissue culture plates at a density of 10$^5$ cells per well. Cells are washed once by replacing media with DMEM and 0.2% BSA before adding PE (100 ng/ml) or PE (100 ng/ml) with extracts which contained an excess amount of different sizes of PE (Table 2). After 15 minutes at 37 °C, the medium is then removed and replaced with fresh DMEM and 0.2% BSA. Four hours later, the rate of protein synthesis is assayed by adding [$^3$H] leucine to the medium (to a final concentration of 2-4 uCi/ml) for 1 hour.

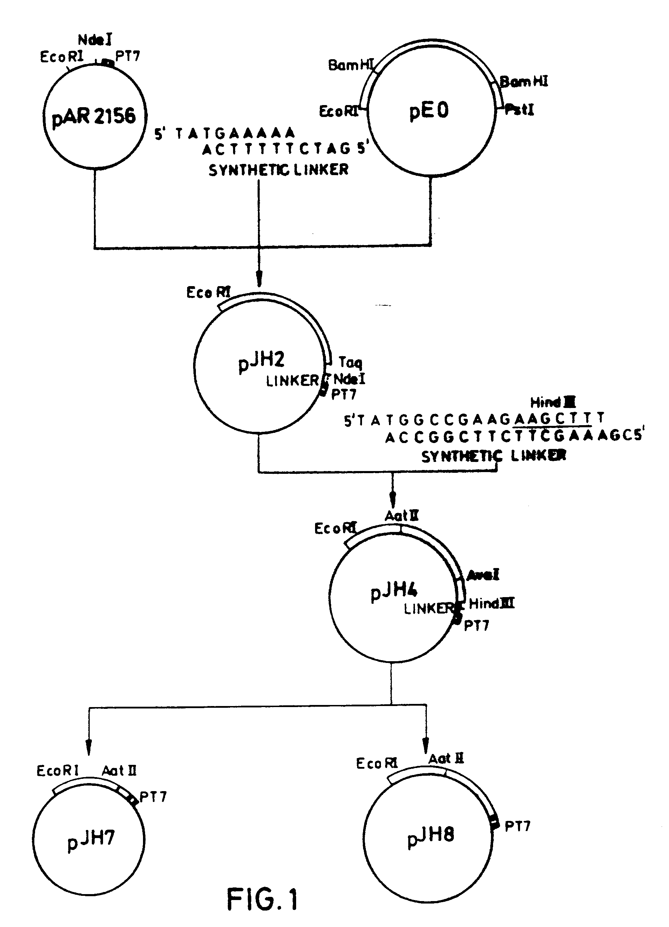

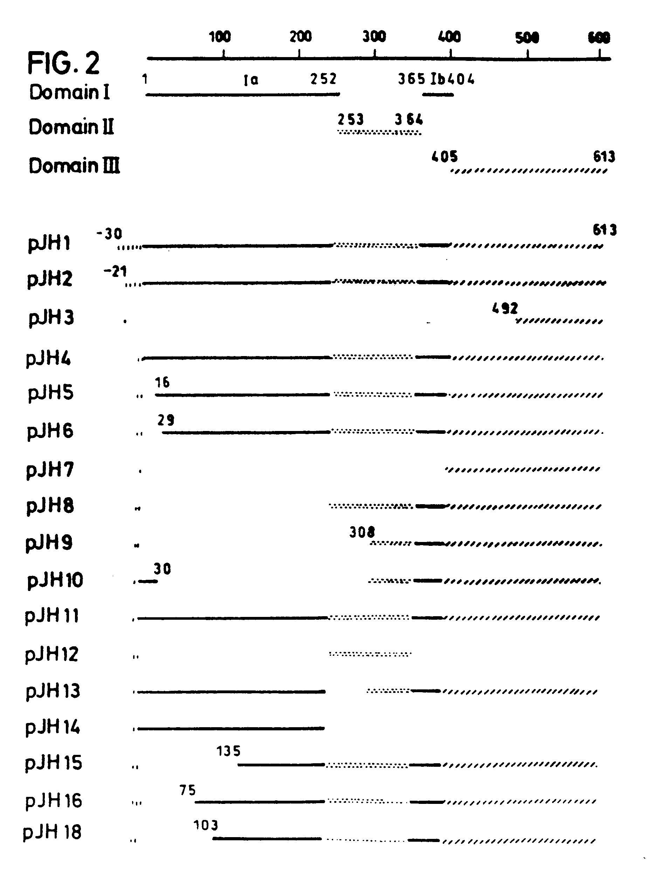

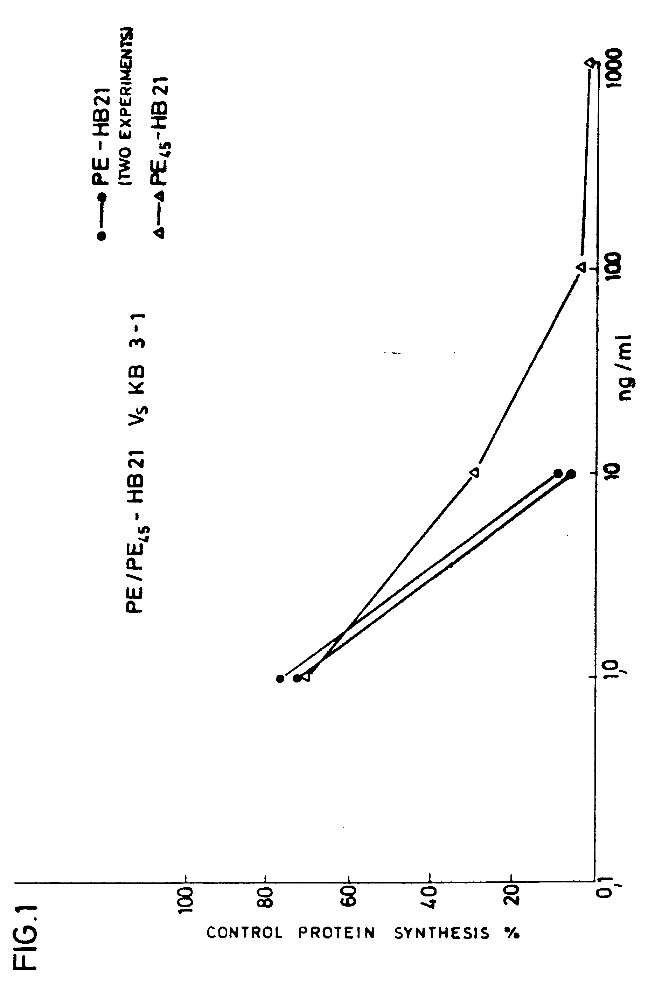

In the preferred embodiment of the invention, the structural domain of the Pseudomonas exotoxin gene is inserted into a T7 expression vector downstream of the ribosome binding site and its accompanying ATG initiation codon (as shown in Figure 1), as described above. To produce recombinant proteins, cells are grown at 37°C to an A$_{650}$ = 0.3. IPTG is then added at 1mM to induce T7 RNA polymerase and incubated for 2 hours. A series of plasmids are produced, as shown in Example 1.

Next, a clone is constructed which encodes a PE molecule without a leader sequence (pJH4) and in which a methionine is placed adjacent to the alanine at the amino terminus of the processed form of native PE (Table 1). The protein produced by pJH4 is designated Met-PE. Large amounts of Met-PE are produced upon induction by IPTG, representing 20% of total cell protein. ADP ribosylating activity equivalent to 0.1 mg native PE is found in the supernatant, and 0.2 mg in the pellet per mg of total cell protein. The PE molecule produced by pJH4 differs from native PE molecules by the presence of one extra methionine residue at the amino-terminus.

pJH8, produced from pJH4 as noted above, expresses a protein in which most of Domain Ia is deleted (retaining only the added methionine and three amino acids at the amino terminus. Immunoblotting shows that this protein is 45 kd, and is expressed at a concentration of approximately 0.04 mg/mg cell protein. High ADP ribosylating activity is exhibited when urea and DTT is excluded from the reaction mixture. In contrast to native PE (where the addition of urea and DTT activates ADP ribosylating activity), the addition of urea and DTT to pJH8 reduces, (by about 30%) ADP ribosylating activity.

None of the plasmids produced by the above-noted process (and described in the Examples), except for pJH1, pJH2, and pJH4, exhibit significant cell killing ability, even though many of these exhibit high enzymatic activity (Table 1). These plasmids are shown to prevent inhibition of protein synthesis or cell killing by native PE by exposing cells for 15 minutes to native PE at 0.1 ug/ml in the presence of 3-5 ug/ml of various modified toxins. The data in Table III shows that plasmids expressing either Domain Ia alone, or Domain I, half of Domain II and Domain III prevent PE from inhibiting protein synthesis.

Animal Toxicity

Mice receiving native PE succumb due to liver destruction. The lethal dose for a 20 gram mouse is 0.1-0.2 ug. The data in Table IV shows that PE with a deletion of Domain Ia exhibits greatly diminished toxicity in mice. Mice were injected I.P. with either native PE or lacking Domain Ia. All mice receiving 1.0 ug of native PE and one-half receiving 0.2 ug PE were dead at 48 hours. Two of three mice receiving 50 ug of PE Ia died; all receiving 20 ug of PE Ia lived; and all mice receiving 5 ug PE Ia lived. Thus, PE Ia is more than 100 times less toxic on a weight basis than native PE.

Gene Fusions Using Recombinant Modified Pseudomonas Exotoxin.

The gene for modified PE of this invention specifically contained in pJH8 was fused with DNA sequences encoding the human alpha transforming growth factor (a-TGF) or human interleukin 2 (IL-2). See Examples 7 and 8 for the specifics of these gene fusions. Modified PE is suitable for use in fusions with any peptide hormone, growth factor, or other polypeptide cell recognition protein for which a specific receptor exists on cells. A few examples include: insulin, glucagon, endorphins, growth hormone, melanocyte stimulating hormone, transferrin, bombesin, low density lipoprotein, luteinizing hormone, and asialoglycoproteins.

Examples

Example 1. As shown in Table 1, the process of the present invention was used to construct several plasmids containing fusions of the different sizes of PE structural gene and the T7 late promoter. pJH2 contains the intact PE structural gene with a segment coding for a modified leader sequence in front. pJH4 contains the intact PE structural gene with the addition of a methionine codon at the amino-terminus. pJH7 contains the gene encoding structural Domain III of PE (amino acid 405-613). pJH8 contains the gene encoding structural Domain II, Ib, and III-of PE (amino acid 253-613). pJH13 encodes PE with a deletion of the first half of structural domain II encompassing amino acids 253-307. pJH14 encodes structural domain Ia of PE (amino acids 1-252).

pJH1 was constructed by partially cutting the native PE (pE 0) with BamHI, and the linear form of DNA was eluted and completely cut with EcoRI. The 2.0kb DNA fragment derived from pE 0, which has BamHI and EcoRI at the two ends, was then inserted into pAR 2156, which had been completely cut with BamHI and EcoRI.

pJH2 was constructed by partially cutting pJH1 with BamHI. The linear form of DNA was isolated and completely cut with NdeI. The 610 kb DNA fragment was saved to construct pJH2 by ligating it with synthetic oligonucleotide duplex

6

```
5' T A T G A A A A A
        A C T T T T T C T A G 5'
```

pJH4 was constructed by partially cutting pJH2 with TaqI. The linearized DNA (610 kb) was isolated and completely cut with NdeI. The largest DNA fragment (5.9 kb) was separated and ligated with synthetic oligonucleotide duplex

```
5' T A T G G C C G A A G A A G C T T T
        A C C G G C T T C T T C G A A A G C 5'
```

**(which contains a HindIII site--AAGCTT).**
                                **TTCGAA**

pJH7 was constructed by partially cutting pJH4 with AatII. The linearized DNA fragment (5.9 kb) was then completely cut with HindIII. The 4.7 kb DNA fragment which has AatII and HindIII sites at its ends after separation was incubated with S1 nuclease to remove the cohesive ends, followed by ligation with T4 ligase.

pJH8 was constructed as described in the Specific Disclosure.

pJH13 was constructed by partially cutting pJH4 with AvaI. The linearized DNA fragment was then completely cut with EcoRI. The 4.7 kb DNA which has AvaI and EcoRI cut at both ends was incubated with S1 to remove cohesive ends (DNA fragment 1). pJH4 was partially cut with SalI. The linearized DNA fragment was then completely cut with EcoRI. The 1.0 kb DNA fragment which has SalI and EcoRI sites at the ends was incubated with Klenow DNA polymerase I and dNTP to fill the cohesive ends (DNA fragment 2). DNA fragment 1 (4.7 kb) and DNA fragment 2 (1.0 kb) were ligated at 4° overnight.

pJH14 was constructed by partially cutting pJH4 with AvaI. The linearized DNA fragment was then completely cut with EcoRI. The 4.7 kb DNA which has AvaI and EcoRI sites at its ends was incubated with S1 to remove the cohesive end, followed by ligation with T4 ligase.

Example 2. The amount and activity of the recombinant toxins (produced by the plasmids described in Example 1) were measured by SDS-PAGE, ADP-ribosylation, and cell killing ability. The results are tabulated in Table 1.

Example 3. Experiments were conducted to examine the characteristics of the recombinant toxicity of the present invention. The results are shown in Table 2.

Example 4. The effect of various deletions on cell protein synthesis was determined, in the presence and absence of native PE. The results are shown in Table 3. Structural Domain Ia (pJH14) and structural domain I, half of II, and III (pJH13) were extracted from the pellet of the sonicated cells with 8M urea. 10 ul of each extract equivalent to 3-5 ug of recombinant proteins was used in each assay. Structural II, Ib, and III (pJH8) were present in the supernatant of the sonicated BL21(DE$_3$)/pJH8 cells. 10 ul of extract equivalent to 2 ug of recombinant toxin was used. Cells were treated as indicated in Table 3 for 15 minutes with and without native PE at 0.1 ug/ml followed by 1 ml DMEM washing; incubated for 4 hours in DMEM and 0.2% BSA, then incubated with $^3$H-leucine for 1 hour.

Example 5. As shown in Table 4, the dose causing death in mice was determined by injecting Balb/c mice I.P. with various amounts of PE or PE Ia contained in 1.0 ml of sterile saline and 10 mgs/ml sterile human albumin. The animals were monitored daily for two weeks. All deaths occured at 48 hours.

Example 6. As shown in Figure 3, an immunotoxin composed of the 45 kD protein produced by pJH8 conjugated by a disulfide bond to an antibody to the human transferrin receptor (PE$_{45}$-HB21) kills human cells expressing the transferrin receptor (ID$_{50}$ 3 ng/ml). It has little or no effect on mouse cells which do not express the human transferrin receptor at 1000 ng/ml, whereas native PE conjugated by a sulfide bond to HB21 nonspecifically kills mouse cells at an ID$_{50}$ of 29 ng/ml. The data in Figure 3 indicate the non-specific toxicity of PE$_{45}$-HB21 is 100-fold less than PE-HB21.

The molecular weight of PE$_{45}$ was subsequently re-determined. The molecular weight was then found to be 40,000.

Example 7. Construction of alpha Transforming Growth Factor-PE fusion gene. pJH8 was treated with Tth IIII and SphI to construct a smaller plasmid pVC8 which has fewer AvaII sites. pVC8 was partially cut with AvaII and ligated to a synthetic oligonucleotide (30bp) which contains a STuI site, a Tth IIII site, and a

stop codon in order to create pVC31. pVC31 was cut with Tth IIII, and filled in with a Klenow fragment, a fragment of DNA polymerase, and ligated to a blunt ended clone containing the alpha-TGF gene (pVC 33). The alpha-TGF DNA, p-hTGF-10-925 [Derynck etal, Cell, 38:287-297 (1984)] was cut with EcoRI and BglI to give a 322 bp fragment which was isolated and cut with Fnu 4HI and treated with T4 polymerase to give a 152 bp fragment which in turn was ligated to pVC31 to create PE-alphaTGF fusion gene. When expressed in E. coli B121, this plasmid produces a protein that reacts with antibodies to alpha-TGF and to PE and has a molecular weight of 51,000.

Example 8. Construction of IL-2-PE Fusion Gene.

pVC8 was cut with AvaII at position 1190, the 3.6 fragment isolated, its single stranded ends filled with Klenow enzyme, and dephosphorylated to produce fragment I. Clone PST-5 [Gallo et al, PNAS, 81:2543-2547 (1984)] was cut with PstI to produce a 1 kb fragment of IL-2 which was then cut with Bsp 1286 at positions 105 and 669 and treated with T4 polymerase to fill up the ends. The 564 bp fragment was ligated to fragment I to create pHL-1 and transformed into BL21 expression cells. Upon induction, a 60 kD protein was produced that reacts with antibodies to IL-2 and to PE, and contains ADP-ribosylating activity.

EP 0 261 671 B1

## TABLE 1

### Summary of the Amount and Activity of the Recombinant Toxins Measured By SDS-PAGE, ADP-ribosylation, and Cell Killing Experiments

#### Amount of PE per mg of Cellular Protein

|  | Measured by SDS-PAGE (mg) | Measured by ADP-ribosylation* (units) |  | Measured by Cell Killing (units) |  |
|---|---|---|---|---|---|
|  | Total | Sup. | Pellet | Sup. | Pellet |
| pJH1 | $0.20^a$ | N.D. | N.D. | N.D. | N.D. |
| pJH2 | $0.20^a$ | N.D. | N.D. | N.D. | N.D. |
| pJH3 | degraded | <0.001 | <0.001 | N.D. | N.D. |
| pJH4 | $0.20^a$ | 0.10 | 0.20 | <0.001 | 0.2 |
| pJH5 | degraded | 0.15 | 0.02 | N.D. | N.D. |
| pJH6 | degraded | <0.001 | <0.001 | N.D. | N.D. |
| pJH7 | $0.25^{o,a}$ | <0.001 | <0.001 | <0.001 | <0.001 |
| pJH8 | $0.04^{o,n}$ | 0.66 | 0.04 | <0.001 | <0.001 |
| pJH9 | $0.15^a$ | 0.40 | 0.20 | <0.001 | <0.001 |
| pJH10 | $0.15^a$ | 0.40 | 0.15 | <0.001 | 0.001 |
| pJH11 | $0.25^a$ | <0.001 | <0.001 | <0.001 | <0.001 |
| pJH12 | $<0.01^{o,n}$ | <0.001 | <0.001 | N.D. | N.D. |
| pJH13 | 0.01 | 0.25 | 0.20 | <0.001 | <0.001 |
| pJH14 | $0.30^a$ | <0.001 | <0.001 | <0.001 | <0.001 |
| pJH15 | $0.10^a$ | 0.18 | 0.30 | <0.001 | <0.001 |
| pJH16 | $<0.01^n$ | 0.02 | N.D. | N.D. | N.D. |
| pJH18 | $<0.01^n$ | 0.02 | N.D. | N.D. | N.D. |

* 1 unit of ADP-ribosylation or cell killing activity is equivalent to the activity from 1 mg of native PE.

N.D. = not determined     a = aggregated     o = positive by Western     n = not visible on SDS PAGE

## TABLE 2

| Construction | Domain Present | Protein Size |
|---|---|---|
| pJH4 | met, I, II, III | 68 kd |
| pJH7 | III | 28 kd |
| pJH8 | II, Ib, III | 45 kd |
| pJH13 | I, half of II, III | 63 kd |
| pJH14 | Ia | 32 kd |

## TABLE 3

$^3$H-leucine in corporation (cpm x $10^3$)

| Domain Tested | without PE | with PE (I, II, III) |
|---|---|---|
| none | $11.2 \pm .1$ | $2.3 \pm .2$ |
| Ia | $11.5 \pm .15$ | $9.4 \pm .3$ |
| II, Ib, & III | $11.7 \pm .15$ | $2.4 \pm .2$ |
| I, 1/2 II, III | $10.7 \pm .15$ | $9.2 \pm .2$ |
| 8 M urea (10 ul) | $11.1 \pm .1$ | $2.9 \pm .2$ |

## TABLE 4

| Toxin | Dose (ug) | Deaths |
|---|---|---|
| $PE_{45}$ | 50 | 2/3 |
| $PE_{45}$ | 20 | 0/3 |
| $PE_{45}$ | 5 | 0/3 |
| $PE_{45}$ | 10 | 3/3 |
| PE | 1.0 | 3/3 |
| PE | 0.3 | 3/3 |
| PE | 0.2 | 1/3 |
| PE | 0.1 | 0/3 |

Statement of Deposit

The following plasmids have been deposited in the American Type Culture Collection in Rockville, Maryland, under the respective ATCC numbers, prior to the filing of this application, and at issuance of this application into a patent will be maintained for a term of thirty (30) years from the date of deposit or five (5) years after the last request for such deposit or for the effective life of the patent, whichever is longest. The deposits will be replaced if the cultures mutate or become nonviable during the term of the deposit:

pJH12    ATCC 67205

PHL-1     ATCC 67206
pVC33     ATCC 67207
pJH8      ATCC 67208
pJH14     ATCC 67209

**Claims**
**Claims for the following Contracting States : BE, CH, DE, FR, GB, IT, LI, LU, NL, SE**

1.  A modified Pseudomonas exotoxin which comprises ADP ribosylating activity and the ability to translocate across a cell membrane and wherein the exotoxin comprises a modification in the receptor binding domain Ia of the native toxin sufficient to render the modified toxin less toxic to human or animal cells in vitro and less toxic to the liver when administered in vivo relative to an unmodified Pseudomonas exotoxin.

2.  A targeting carrier-toxin conjugate suitable for use with humans or mammals comprising a toxin fused to a targeting carrier wherein said toxin is a modified Pseudomonas exotoxin according to claim 1.

3.  The conjugate of claim 2, wherein said targeting carrier is selected from the group consisting of an antigen, an antibody, a growth factor, a hormone, a lymphokine, a polypeptide cell recognition protein, a serum protein, a modified serum protein, an endorphin, an asialoglycoprotein, insulin, glucagon, luteinizing hormone, fibroblast growth factor, nerve growth factor, melanocyte stimulating hormone, bombesin, and a lectin.

4.  The conjugate of claim 2, wherein said targeting carrier is selected from the group consisting of interleukin 2 and alpha transforming growth factor and a peptide hormone.

5.  An immunotoxin suitable for use with humans or animals comprising a toxin fused to an antibody capable of recognizing diseased or disease-causing cells wherein said toxin is a modified Pseudomonas exotoxin according to claim 1.

6.  The immunotoxin of claim 5, wherein said exotoxin contains domains Ib, II and III of Pseudomonas exotoxin.

7.  The immunotoxin of claim 5, wherein said exotoxin is the recombinant Pseudomonas exotoxin encoded by plasmid pJH8 deposited under ATCC number 67208.

8.  A Pseudomonas immunotoxin according to claim 5, wherein at least one amino acid is deleted from domain Ia of said exotoxin.

9.  A Pseudomonas immunotoxin according to claim 5, wherein at least 11 amino acids in domain Ia have been deleted from said exotoxin.

10. A Pseudomonas immunotoxin according to claim 5, wherein at least 25 amino acids in domain Ia have been deleted from said exotoxin.

11. A Pseudomonas immunotoxin according to claim 5, wherein at least 70 amino acids in domain Ia have been deleted from said exotoxin.

12. A Pseudomonas immunotoxin according to claim 5, wherein at least 130 amino acids from domain Ia have been deleted from said exotoxin.

13. An expression plasmid comprising DNA encoding any of the proteins of claims 1 to 12.

14. A process for producing a modified Pseudomonas exotoxin according to claim 1 comprising transfecting a suitable host cell with a plasmid comprising DNA encoding said modified exotoxin and culturing said host cell under conditions so that the modified Pseudomonas exotoxin is produced.

15. A process for producing an immunotoxin according to claim 5 comprising

11

a) transfecting a suitable host cell with a plasmid encoding the modified Pseudomonas exotoxin, according to claim 1. b) culturing said host cell under conditions so
that the modified Pseudomonas exotoxin is produced, and
c) conjugating said modified exotoxin with an antibody to produce an immunotoxin.

16. The process of claim 15 wherein said modified Pseudomonas exotoxin gene contains domains Ib, II and III of Pseudomonas exotoxin.

17. A process for producing a targeting carrier Pseudomonas exotoxin conjugate according to claim 2, comprising transfecting a suitable host cell with a plasmid comprising a DNA sequence encoding said targeting carrier conjugate, and culturing said host cell under conditions so that a targeting carrier-Pseudomonas exotoxin conjugate is produced.

18. A composition suitable for administration to a mammal for achieving targeted cytotoxic activity in said mammal, wherein the composition comprises a conjugate according to any one of claims 2 to 12 in a pharmaceutically acceptable carrier.

19. A recombinant plasmid comprising DNA encoding amino acids 253 through 364 of domain II of Pseudomonas exotoxin.

20. The recombinant plasmid of claim 19, wherein the plasmid is pJH12 deposited under ATCC number 67205.

21. A recombinant plasmid comprising DNA encoding a modified Pseudomonas exotoxin according to claim 1 fused to DNA encoding at least amino acids 35 through 223 of interleukin 2.

22. The recombinant plasmid of claim 21 wherein the plasmid is pHL-1 deposited under ATCC number 67206.

23. A recombinant plasmid comprising DNA encoding a modified Pseudomonas exotoxin according to claim 1 fused to DNA encoding alpha TGF.

24. The recombinant plasmid of claim 23 wherein the plasmid is pVC33 deposited under ATCC number 67207.

25. A recombinant plasmid comprising DNA encoding a modified Pseudomonas exotoxin according to claim 1.

26. The recombinant plasmid of claim 25 wherein the plasmid is pJHB deposited under ATCC number 67208.

27. A recombinant plasmid comprising DNA encoding structural domain Ia of Pseudomonas exotoxin.

28. The recombinant plasmid of claim 27 wherein the plasmid is pJH14 deposited under ATCC number 67209.

29. A modified Pseudomonas exotoxin according to claim 1 wherein said exotoxin has been genetically modified.

30. An antigen-toxin conjugate according to claim 3 wherein said exotoxin has been modified by recombinant techniques.

31. Use of an exotoxin according to claim 1 or of an exotoxin encoded by any of the plasmids of claims 19 to 28 for the preparation of a vaccine for active immunization.

32. Use of a conjugate according to any of claims 2 to 12 for the preparation of a medicament for treating cancer.

EP 0 261 671 B1

**Claims for the following Contracting States : ES, AT**

1. A process for producing a modified Pseudomonas exotoxin which comprises ADP ribosylating activity and the ability to translocate across a cell membrane and wherein the exotoxin comprises a modification in the receptor binding domain Ia of the native toxin sufficient to render the modified toxin less toxic to human or animal cells in vitro and less toxic to the liver when administrered in vivo relative to an unmodified Pseudomonas exotoxin, comprising transfecting a suitable host cell with a plasmid comprising DNA encoding said modified exotoxin and culturing said host cell under conditions so that the modified Pseudomonas exotoxin is produced.

2. A process for producing a targeting carrier toxin conjugate suitable for use with humans or mammals which comprises a toxin fused to a targeting carrier wherein said toxin is a modified Pseudomonas exotoxin according to claim 1, comprising transfecting a suitable host cell with a plasmid comprising DNA encoding said modified exocoxin and culturing said host cell under conditions so that the modified Pseudomonas exotoxin is produced.

3. The process or claim 2, wherein said targeting carrier is selected from the group consisting of an antigen, an antibody, a growth factor, a hormone, a lymphokine, a polypeptide cell recognition protein, a serum protein, a modified serum protein, an endorphin, an asialoglycoprotein, insulin, glucagon, luteinizing hormone, fibroblast growth factor, nerve growth factor, melanocyte stimulating hormone, bombesin, and a lectin.

4. The process or claim 2, wherein said targeting carrier is selected from the group consisting of interleukin 2 and alpha transforming growth factor and a peptide hormone.

5. A process for producing an immunotoxin suitable for use with humans or animals which comprises a toxin fused to an antibody capable of recognizing diseased or disease-causing cells wherein said toxin is a modified Pseudomonas exotoxin according to claim 1, comprising
   a) transfecting a suitable host cell with a plasmid encoding the modified Pseudomonas exotoxin according to claim 1.
   b) culturing said host cell under condition so that the modified Pseudomonas exotoxin in produced, and
   c) conjugating said modified exotoxin with an antibody to produce an immunotoxin.

6. The process of claim 5, wherein said modified Pseudomonas exotoxin gene contains domains Ib, II and III of Pseudomonas exotoxin.

7. The process of claim 5, wherein said plasmid is pJH8 deposited under ATCC number 67208.

8. The process of claim 5, wherein at least one amino acid is deleted from domain Ia of said exotoxin.

9. The process of claim 5, wherein at least 11 amino acids in domain Ia have been deleted from said exotoxin.

10. The process of claim 5, wherein at least 25 amino acids in domain Ia have been deleted from said exotoxin.

11. The process of claim 5, wherein at least 70 amino acids in domain Ia have been deleted from said exotoxin.

12. The process of claim 5, wherein at least 130 amino acids from domain Ia have been deleted from said exotoxin.

13. A process for preparing an expression plasmid which comprises DNA encoding any of the proteins of claims 1 to 12, comprising constructing said expression plasmid in a manner known per se.

14. A process for preparing a composition suitable for administration to a mammal for achieving targeted cytotoxic activity in said mammal, wherein the composition comprises a conjugate according to any

13

EP 0 261 671 B1

one of claims 2 to 12 in a pharmaceutically acceptable carrier, comprising mixing said conjugate and said pharmaceutically acceptable carrier in a manner known per se.

15. A process for preparing a recombinant plasmid which comprises DNA encoding amino acids 253 through 364 of domain II of Pseudomonas exotoxin, comprising constructing said recombinant plasmid in a manner known per se.

16. The process of claim 15, wherein the plasmid is pJH12 deposited under ATCC number 67205.

17. A process for preparing a recombinant plasmid which comprises DNA encoding a modified Pseudomonas exotoxin according to claim 1 fused to DNA encoding at least amino acids 35 through 223 of interleukin 2, comprising constructing said recombinant plasmid in a manner known per se.

18. The process of claim 17 wherein the plasmid is pHL-1 deposited under ATCC number 67206.

19. A process for preparing a recombinant plasmid which comprises DNA encoding a modified Pseudomonas exotoxin according to claim 1 fused to DNA encoding alpha TGF, comprising constructing said recombinant plasmid in a manner known per se.

20. The process of claim 19 wherein the plasmid is pVC33 deposited under STCC number 67207.

21. A process for preparing a recombinant plasmid which comprises DNA encoding a modified Pseudomonas exotoxin according to claim 1, comprising constructing said recombinant plasmid in a manner known per se.

22. The process of claim 21 wherein the plasmid is pJH8 deposited under ATCC number 67208.

23. A process for preparing a recombinant plasmid which comprises DNA encoding structural domain Ia of Pseudomonas exotoxin, comprising constructing said recombinant plasmid in a manner known per se.

24. The process of claim 23 wherein the plasmid is pJH14 deposited under ATCC number 67209.

25. A process for preparing a modified Pseudomonas exotoxin according to claim 1 comprising modifying said exotoxin genetically.

26. A process for preparing an antigen-toxin conjugate according to claim 3 comprising modifying said exotoxin by recombinant techniques.

27. A process for preparing a vaccine for active immunization which comprises an exotoxin obtained according to claim 1 or an exotoxin encoded by any of the plasmids according to claims 15 to 24 comprising formulating said vaccine in a manner known per se.

28. A process for preparing a medicament for treating cancer which comprises a conjugate obtained according to any of claims 2 to 12 comprising formulating said medicament in a manner known per se.

**Claims for the following Contracting State : GR**

1. A modified Pseudomonas exotoxin which comprises ADP ribosylating activity and the ability to translocate across a cell membrane and wherein the exotoxin comprises a modification in the receptor binding domain Ia of the native toxin sufficient to render the modified toxin less toxic to human or animal cells in vitro and less toxic to the liver when administered in vivo relative to an unmodified Pseudomonas exotoxin.

2. A targeting carrier-toxin conjugate suitable for use with humans or mammals comprising a toxin fused to a targeting carrier wherein said toxin is a modified Pseudomonas exotoxin according to claim 1.

3. The conjugate of claim 2, wherein said targeting carrier is selected from the group consisting of an antigen, an antibody, a growth factor, a hormone, a lymphokine, a polypeptide cell recognition protein,

14

a serum protein, a modified serum protein, an endorphin, an asialoglycoprotein, insulin, glucagon, luteinizing hormone, fibroblast growth factor, nerve growth factor, melanocyte stimulating hormone, bombesin, and a lectin.

4. The conjugate of claim 2, wherein said targeting carrier is selected from the group consisting of interleukin 2 and alpha transforming growth factor and a peptide hormone.

5. An immunotoxin suitable for use with humans or animals comprising a toxin fused to an antibody capable of recognizing diseased or disease-causing cells wherein said toxin is a modified Pseudomonas exotoxin according to claim 1.

6. The immunotoxin of claim 5, wherein said exotoxin contains domains Ib, II and III of Pseudomonas exotoxin.

7. The immunotoxin of claim 5, wherein said exotoxin is the recombinant Pseudomonas exotoxin encoded by plasmid pJH8 deposited under ATCC number 67208.

8. A Pseudomonas immunotoxin according to claim 5, wherein at least one amino acid is deleted from domain Ia of said exotoxin.

9. A Pseudomonas immunotoxin according to claim 5, wherein at least 11 amino acids in domain Ia have been deleted from said exotoxin.

10. A Pseudomonas immunotoxin according to claim 5, wherein at least 25 amino acids in domain Ia have been deleted from said exotoxin.

11. A Pseudomonas immunotoxin according to claim 5, wherein at least 70 amino acids in domain Ia have been deleted from said exotoxin.

12. A Pseudomonas immunotoxin according to claim 5, wherein at least 130 amino acids from domain Ia have been deleted from said exotoxin.

13. An expression plasmid comprising DNA encoding any of the proteins of claims 1 to 12.

14. A process for producing a modified Pseudomonas exotoxin according to claim 1 comprising transfecting a suitable host cell with a plasmid comprising DNA encoding said modified exotoxin and culturing said host cell under conditions so that the modified Pseudomonas exotoxin is produced.

15. A process for producing an immunotoxin according to claim 5 comprising
    a) transfecting a suitable host cell with a plasmid encoding the modified Pseudomonas exotoxin, according to claim 1.
    b) culturing said host cell under conditions so that the modified Pseudomonas exotoxin is produced, and
    c) conjugating said modified exotoxin with an antibody to produce an immunotoxin.

16. The process of claim 15 wherein said modified Pseudomonas exotoxin gene contains domains Ib, II and III of Pseudomonas exotoxin.

17. A process for producing a targeting carrier Pseudomonas exotoxin conjugate according to claim 2, comprising transfecting a suitable host cell with a plasmid comprising a DNA sequence encoding said targeting carrier conjugate, and culturing said host cell under conditions so that a targeting carrier-Pseudomonas exotoxin conjugate is produced.

18. A process for preparing a composition suitable for administration to a mammal for achieving targeted cytotoxic activity in said mammal, wherein the composition comprises a conjugate according to any one of claims 2 to 12 in a pharmaceutically acceptable carrier, comprising mixing said conjugate and said pharmaceutically acceptable carrier in a manner known per se.

15

**19.** A recombinant plasmid comprising DNA encoding amino acids 253 through 364 of domain II of Pseudomonas exotoxin.

**20.** The recombinant plasmid of claim 22, wherein the plasmid is pJH12 deposited under ATCC number 67205.

**21.** A recombinant plasmid comprising DNA encoding a modified Pseudomonas exotoxin according to claim 1 fused to DNA encoding at least amino acids 35 through 223 of interleukin 2.

**22.** The recombinant plasmid of claim 21 wherein the plasmid is pHL-1 deposited under ATCC number 67206.

**23.** A recombinant plasmid comprising DNA encoding a modified Pseudomonas exotoxin according to claim 1 fused to DNA encoding alpha TGF.

**24.** The recombinant plasmid of claim 23 wherein the plasmid is pVC33 deposited under ATCC number 67207.

**25.** A recombinant plasmid comprising DNA encoding a modified Pseudomonas exotoxin according to claim 1.

**26.** The recombinant plasmid of claim 25 wherein the plasmid is pJH8 deposited under ATCC number 67208.

**27.** A recombinant plasmid comprising DNA encoding structural domain Ia of Pseudomonas exotoxin.

**28.** The recombinant plasmid of claim 27 wherein the plasmid is pJH14 deposted under ATCC number 67209.

**29.** A modified Pseudomonas exotoxin according to claim 1 wherein said exotoxin has been genetically modified.

**30.** An antigen-toxin conjugate according to claim 3 wherein said exotoxin has been modified by recombinant techniques.

**31.** Use of an exotoxin according to claim 1 or of an exotoxin encoded by any of the plasmids of claims 19 to 28 for the preparation of a vaccine for active immunization.

**32.** Use of a conjugate according to any of claims 2 to 12 for the preparation of a medicament for treating cancer.

**Patentansprüche**
**Patentansprüche für folgende Vertragsstaaten : BE, CH, DE, FR, GB, IT, LI, LU, NL, SE**

**1.** Modifiziertes Pseudomonas-Exotoxin, welches ADP-Ribosylierungsaktivität und die Fähigkeit, eine Zellmembran zu passieren, umfaßt, und worin das Exotoxin eine Modifizierung in der Rezeptor-bindenden Domäne Ia des nativen Toxins umfaßt, die ausreichend ist, das modifizierte Toxin weniger toxisch für humane oder tierische Zellen in vitro und weniger toxisch für die Leber im Vergleich zu einem nicht-modifizierten Pseudomonas-Exotoxin werden zu lassen, wenn dieses in vivo verabreicht wird.

**2.** Konjugat aus einem zielorientierten Träger und Toxin, welches für die Verwendung bei Menschen oder Säugetieren geeignet ist, umfassend ein Toxin, welches an einen zielorientierten Träger fusioniert ist, worin das Toxin ein modifiziertes Pseudomonas-Exotoxin nach Anspruch 1 ist.

**3.** Konjugat nach Anspruch 2, worin der zielorientierte Träger ausgewählt ist aus der Gruppe, bestehend aus einem Antigen, einem Antikörper, einem Wachstumsfaktor, einem Hormon, einem Lymphokin, einem Polypeptid-Zellerkennungsprotein, einem Serumprotein, einem modifizierten Serumprotein, einem Endorphin, einem Asialoglykoprotein, Insulin, Glucagon, luteinisierendem Hormon, Fibroblastenwachstumsfaktor, Nervenwachstumsfaktor, Melanocyten-stimulierendem Hormon, Bombesin und einem Lectin.

4. Konjugat nach Anspruch 2, worin der zielorientierte Träger ausgewählt ist aus der Gruppe, bestehend aus Interleukin 2 und α-transformierendem Wachstumsfaktor und einem Peptidhormon.

5. Immuntoxin, welches für die Verwendung bei Menschen oder Tieren geeignet ist, umfassend ein Toxin, welches an einen Antikörper fusioniert ist, der fähig ist, erkrankte oder Krankheit verursachende Zellen zu erkennen, worin das Toxin ein modifiziertes Pseudomonas-Exotoxin nach Anspruch 1 ist.

6. Immuntoxin nach Anspruch 5, worin das Exotoxin die Domänen Ib, II und III des Pseudomonas-Exotoxins enthält.

7. Immuntoxin nach Anspruch 5, worin das Exotoxin das rekombinante Pseudomonas-Exotoxin ist, welches durch das Plasmid pJH8 kodiert ist, das unter der ATCC-Nr. 67208 hinterlegt ist.

8. Pseudomonas-Immuntoxin nach Anspruch 5, worin mindestens eine Aminosäure aus der Domäne Ia des Exotoxins deletiert ist.

9. Pseudomonas-Immuntoxin nach Anspruch 5, worin mindestens 11 Aminosäuren in der Domäne Ia aus dem Exotoxin deletiert worden sind.

10. Pseudomonas-Immuntoxin nach Anspruch 5, worin mindestens 25 Aminosäuren in der Domäne Ia aus dem Exotoxin deletiert worden sind.

11. Pseudomonas-Immuntoxin nach Anspruch 5, worin mindestens 70 Aminosäuren in der Domäne Ia aus dem Exotoxin deletiert worden sind.

12. Pseudomonas-Immuntoxin nach Anspruch 5, worin mindestens 130 Aminosäuren aus der Domäne Ia aus dem Exotoxin deletiert worden sind.

13. Expressionsplasmid, umfassend DNA, welche eines der Proteine nach den Ansprüchen 1 bis 12 kodiert.

14. Verfahren zum Herstellen eines modifizierten Pseudomonas-Exotoxins nach Anspruch 1, umfassend das Transfizieren einer geeigneten Wirtszelle mit einem Plasmid, umfassend DNA, welche das modifizierte Exotoxin kodiert, und das Kultivieren der Wirtszelle unter Bedingungen, so daß das modifizierte Pseudomonas-Exotoxin produziert wird.

15. Verfahren zum Herstellen eines Immuntoxins nach Anspruch 5, umfassend
a) das Transfizieren einer geeigneten Wirtszelle mit einem Plasmid, welches das modifizierte Pseudomonas-Exotoxin nach Anspruch 1 kodiert
b) das Kultivieren der Wirtszelle unter Bedingungen, so daß das modifizierte Pseudomonas-Exotoxin produziert wird, und
c) das Konjugieren des modifizierten Exotoxins mit einem Antikörper, um ein Immuntoxin herzustellen.

16. Verfahren nach Anspruch 15, worin das modifizierte Pseudomonas-Exotoxingen die Domänen Ib, II und III des Pseudomonas-Exotoxins enthält.

17. Verfahren zum Herstellen eines Konjugats aus einem zielorientierten Träger und Pseudomonas-Exotoxin nach Anspruch 2, umfassend das Transfizieren einer geeigneten Wirtszelle mit einem Plasmid, umfassend eine DNA-Sequenz, welche das Konjugat mit dem zielorientierten Träger kodiert, und das Kultivieren der Wirtszelle unter Bedingungen, so daß ein Konjugat aus einem zielorientierten Träger und Pseudomonas-Exotoxin produziert wird.

**18.** Zusammensetzung, welche für die Verabreichung an ein Säugetier zum Erreichen einer zielorientierten zytotoxischen Aktivität in dem Säugetier geeignet ist, worin die Zusammensetzung ein Konjugat nach einem der Ansprüche 2 bis 12 in einem pharmazeutisch annehmbaren Träger umfaßt.

**19.** Rekombinantes Plasmid, umfassend DNA, welche die Aminosäuren 253 bis 364 der Domäne II des Pseudomonas-Exotoxins kodiert.

**20.** Rekombinantes Plasmid nach Anspruch 19, worin das Plasmid pJH12 ist, welches unter der ATCC-Nr. 67205 hinterlegt ist.

**21.** Rekombinantes Plasmid, umfassend DNA, welche ein modifiziertes Pseudomonas-Exotoxin nach Anspruch 1 kodiert, fusioniert an DNA, welche mindestens die Aminosäuren 35 bis 223 von Interleukin 2 kodiert.

**22.** Rekombinantes Plasmid nach Anspruch 21, worin das Plasmid pHL-1 ist, welches unter der ATCC-Nr. 67206 hinterlegt ist.

**23.** Rekombinantes Plasmid, umfassend DNA, welche ein modifiziertes Pseudomonas-Exotoxin nach Anspruch 1 kodiert, fusioniert an DNA, welche α-TGF kodiert.

**24.** Rekombinantes Plasmid nach Anspruch 23, worin das Plasmid pVC33 ist, welches unter der ATCC-Nr. 67207 hinterlegt ist.

**25.** Rekombinantes Plasmid, umfassend DNA, welche ein modifiziertes Pseudomonas-Exotoxin nach Anspruch 1 kodiert.

**26.** Rekombinantes Plasmid nach Anspruch 25, worin das Plasmid pJH8 ist, welches unter der ATCC-Nr. 67208 hinterlegt ist.

**27.** Rekombinantes Plasmid, umfassend DNA, welche die strukturelle Domäne Ia des Pseudomonas-Exotoxins kodiert.

**28.** Rekombinantes Plasmid nach Anspruch 27, worin das Plasmid pJH14 ist, welches unter der ATCC-Nr. 67209 hinterlegt ist.

**29.** Modifiziertes Pseudomonas-Exotoxin nach Anspruch 1, worin das Exotoxin genetisch modifiziert worden ist.

**30.** Konjugat aus einem Antigen und Toxin nach Anspruch 3, worin das Exotoxin durch rekombinante Techniken modifiziert worden ist.

**31.** Verwendung eines Exotoxins nach Anspruch 1 oder eines Exotoxins, welches durch eines der Plasmide nach den Ansprüchen 19 bis 28 kodiert ist, für die Herstellung eines Impfstoffes für aktive Immunisierung.

**32.** Verwendung eines Konjugats nach einem der Ansprüche 2 bis 12 für die Herstellung eines Medikaments zum Behandeln von Krebs.

**Patentansprüche für folgende Vertragsstaaten : ES, AT**

**1.** Verfahren zum Herstellen eines modifizierten Pseudomonas-Exotoxins, welches ADP-Ribosylierungsaktivität und die Fähigkeit, eine Zellmembran zu passieren, umfaßt, und worin das Exotoxin eine Modifizierung in der Rezeptor-bindenden Domäne Ia des nativen Toxins umfaßt, die ausreichend ist, das modifizierte Toxin weniger toxisch für humane oder tierische Zellen in vitro und weniger toxisch für die Leber im Vergleich zu einem nicht-modifizierten Pseudomonas-Exotoxin werden zu lassen, wenn dieses in vivo verabreicht wird, umfassend das Transfizieren einer geeigneten Wirtszelle mit einem Plasmid, umfassend DNA, welche das modifizierte Exotoxin kodiert, und das Kultivieren der Wirtszelle unter Bedingungen, so daß das modifizierte Pseudomonas-Exotoxin produziert wird.

18

2. Verfahren zum Herstellen eines Konjugats aus einem zielorientierten Träger und Toxin, welches für die Verwendung bei Menschen oder Säugetieren geeignet ist, welches ein Toxin umfaßt, das an einen zielorientierten Träger fusioniert ist, worin das Toxin ein modifiziertes Pseudomonas-Exotoxin nach Anspruch 1 ist, umfassend das Transfizieren einer geeigneten Wirtszelle mit einem Plasmid, umfassend DNA, welche das modifizierte Exotoxin kodiert, und das Kultivieren der Wirtszelle unter Bedingungen, so daß das modifizierte Pseudomonas-Exotoxin produziert wird.

3. Verfahren nach Anspruch 2, worin der zielorientierte Träger ausgewählt ist aus der Gruppe, bestehend aus einem Antigen, einem Antikörper, einem Wachstumsfaktor, einem Hormon, einem Lymphokin, einem Polypeptid-Zellerkennungsprotein, einem Serumprotein, einem modifizierten Serumprotein, einem Endorphin, einem Asialoglykoprotein, Insulin, Glucagon, luteinisierendem Hormon, Fibroblastenwachstumsfaktor, Nervenwachstumsfaktor, Melanocyten-stimulierendem Hormon, Bombesin und einem Lectin.

4. Verahren nach Anspruch 2, worin der zielorientierte Träger ausgewählt ist aus der Gruppe, bestehend aus Interleukin 2 und $\alpha$-transformierendem Wachstumsfaktor und einem Peptidhormon.

5. Verfahren zur Herstellung eines Immuntoxins, welches für die Verwendung bei Menschen oder Tieren geeignet ist, umfassend ein Toxin, welches an einen Antikörper fusioniert ist, der fähig ist, erkrankte oder Krankheit verursachende Zellen zu erkennen, worin das Toxin ein modifiziertes Pseudomonas-Exotoxin nach Anspruch 1 ist, umfassend
    a) das Transfizieren einer geeigneten Wirtszelle mit einem Plasmid, welches das modifizierte Pseudomonas-Exotoxin nach Anspruch 1 kodiert
    b) das Kultivieren der Wirtszelle unter Bedingungen, so daß das modifizierte Pseudomonas-Exotoxin produziert wird, und
    c) das Konjugieren des modifizierten Exotoxins mit einem Antikörper, um ein Immuntoxin herzustellen.

6. Verfahren nach Anspruch 5, worin das modifizierte Pseudomonas-Exotoxingen die Domänen Ib, II und III des Pseudomonas-Exotoxins enthält.

7. Verfahren nach Anspruch 5, worin das Plasmid pJH8 ist, das unter der ATCC-Nr. 67208 hinterlegt ist.

8. Verfahren nach Anspruch 5, worin mindestens eine Aminosäure aus der Domäne Ia des Exotoxins deletiert ist.

9. Verfahren nach Anspruch 5, worin mindestens 11 Aminosäuren in der Domäne Ia aus dem Exotoxin deletiert worden sind.

10. Verfahren nach Anspruch 5, worin mindestens 25 Aminosäuren in der Domäne Ia aus dem Exotoxin deletiert worden sind.

11. Verfahren nach Anspruch 5, worin mindestens 70 Aminosäuren in der Domäne Ia aus dem Exotoxin deletiert worden sind.

12. Verfahren nach Anspruch 5, worin mindestens 130 Aminosäuren aus der Domäne Ia aus dem Exotoxin deletiert worden sind.

13. Verfahren zum Herstellen eines Expressionsplasmids, welches DNA umfaßt, welche eines der Proteine nach den Ansprüchen 1 bis 12 kodiert, umfassend das Konstruieren des Expressionsplasmids in einer an sich bekannten Weise.

14. Verfahren zum Herstellen einer Zusammensetzung, welche für die Verabreichung an ein Säugetier zum Erreichen einer zielorientierten zytotoxischen Aktivität in dem Säugetier geeignet ist, worin die Zusammensetzung ein Konjugat nach einem der Ansprüche 2 bis 12 in einem pharmazeutisch annehmbaren Träger umfaßt, umfassend das Mischen des Konjugats und des pharmazeutisch annehmbaren Trägers in einer an sich bekannten Weise.

**15.** Verfahren zum Herstellen eines rekombinanten Plasmids, welches DNA umfaßt, die die Aminosäuren 253 bis 364 der Domäne II des Pseudomonas-Exotoxins kodiert, umfassend das Konstruieren des rekombinanten Plasmids in einer an sich bekannten Weise.

**16.** Verfahren nach Anspruch 15, worin das Plasmid pJH12 ist, welches unter der ATCC-Nr. 67205 hinterlegt ist.

**17.** Verfahren zum Herstellen eines rekombinanten Plasmids, welches DNA umfaßt, die ein modifiziertes Pseudomonas-Exotoxin nach Anspruch 1 kodiert, fusioniert an DNA, welche mindestens die Aminosäuren 35 bis 223 von Interleukin 2 kodiert, umfassend das Konstruieren des rekombinanten Plasmids in einer an sich bekannten Weise.

**18.** Verfahren nach Anspruch 17, worin das Plasmid pHL-1 ist, welches unter der ATCC-Nr. 67206 hinterlegt ist.

**19.** Verfahren zum Herstellen eines rekombinanten Plasmids, welches DNA umfaßt, die ein modifiziertes Pseudomonas-Exotoxin nach Anspruch 1 kodiert, fusioniert an DNA, welche α-TGF kodiert, umfassend das Konstruieren des rekombinanten Plasmids in einer an sich bekannten Weise.

**20.** Verfahren nach Anspruch 19, worin das Plasmid pVC33 ist, welches unter der ATCC-Nr. 67207 hinterlegt ist.

**21.** Verfahren zum Herstellen eines rekombinanten Plasmids, welches DNA umfaßt, die ein modifiziertes Pseudomonas-Exotoxin nach Anspruch 1 kodiert, umfassend das Konstruieren des rekombinanten Plasmids in einer an sich bekannten Weise.

**22.** Verfahren nach Anspruch 21, worin das Plasmid pJH8 ist, welches unter der ATCC-Nr. 67208 hinterlegt ist.

**23.** Verfahren zum Herstellen eines rekombinanten Plasmids, welches DNA umfaßt, die die strukturelle Domäne Ia des Pseudomonas-Exotoxins kodiert, umfassend das Konstruieren des rekombinanten Plasmids in einer an sich bekannten Weise.

**24.** Verfahren nach Anspruch 23, worin das Plasmid pJH14 ist, welches unter der ATCC-Nr. 67209 hinterlegt ist.

**25.** Verfahren zum Herstellen eines modifizierten Pseudomonas-Exotoxins nach Anspruch 1, umfassend das genetische Modifizieren des Exotoxins.

**26.** Verfahren zum Herstellen eines Konjugats aus einem Antigen nach Anspruch 3, umfassend das Modifizieren des Exotoxins durch rekombinante Techniken.

**27.** Verfahren zum Herstellen eines Impfstoffes für aktive Immunisierung, welcher ein Exotoxin, erhalten nach Anspruch 1, oder ein Exotoxin, welches durch eines der Plasmide nach den Ansprüchen 15 bis 24 kodiert ist, umfaßt, umfassend das Formulieren des Impfstoffes auf eine an sich bekannte Weise.

**28.** Verfahren zum Herstellen eines Medikaments zum Behandeln von Krebs, welches ein Konjuguat, erhalten nach einem der Ansprüche 2 bis 12 umfaßt, umfassend das Formulieren des Medikaments auf eine an sich bekannte Weise.

**Patentansprüche für folgenden Vertragsstaat : GR**

**1.** Modifiziertes Pseudomonas-Exotoxin, welches ADP-Ribosylierungsaktivität und die Fähigkeit, eine Zellmembran zu passieren, umfaßt, und worin das Exotoxin eine Modifizierung in der Rezeptor-bindenden Domäne Ia des nativen Toxins umfaßt, die ausreichend ist, das modifizierte Toxin weniger toxisch für humane oder tierische Zellen in vitro und weniger toxisch für die Leber im Vergleich zu einem nicht-modifizierten Pseudomonas-Exotoxin werden zu lassen, wenn dieses in vivo verabreicht wird.

**2.** Konjugat aus einem zielorientierten Träger und Toxin, welches für die Verwendung bei Menschen oder Säugetieren geeignet ist, umfassend ein Toxin, welches an einen zielorientierten Träger fusioniert ist, worin das Toxin ein modifiziertes Pseudomonas-Exotoxin nach Anspruch 1 ist.

**3.** Konjugat nach Anspruch 2, worin der zielorientierte Träger ausgewählt ist aus der Gruppe, bestehend aus einem Antigen, einem Antikörper, einem Wachstumsfaktor, einem Hormon, einem Lymphokin, einem Polypeptid-Zellerkennungsprotein, einem Serumprotein, einem modifizierten Serumprotein, einem Endorphin, einem Asialoglykoprotein, Insulin, Glucagon, luteinisierendem Hormon, Fibroblastenwachstumsfaktor, Nervenwachstumsfaktor, Melanocyten-stimulierendem Hormon, Bombesin und einem Lectin.

**4.** Konjugat nach Anspruch 2, worin der zielorientierte Träger ausgewählt ist aus der Gruppe, bestehend aus Interleukin 2 und α-transformierendem Wachstumsfaktor und einem Peptidhormon.

**5.** Immuntoxin, welches für die Verwendung bei Menschen oder Tieren geeignet ist, umfassend ein Toxin, welches an einen Antikörper fusioniert ist, der fähig ist, erkrankte oder Krankheit verursachende Zellen zu erkennen, worin das Toxin ein modifiziertes Pseudomonas-Exotoxin nach Anspruch 1 ist.

**6.** Immuntoxin nach Anspruch 5, worin das Exotoxin die Domänen Ib, II und III des Pseudomonas-Exotoxins enthält.

**7.** Immuntoxin nach Anspruch 5, worin das Exotoxin das rekombinante Pseudomonas-Exotoxin ist, welches durch das Plasmid pJH8 kodiert ist, das unter der ATCC-Nr. 67208 hinterlegt ist.

**8.** Pseudomonas-Immuntoxin nach Anspruch 5, worin mindestens eine Aminosäure aus der Domäne Ia des Exotoxins deletiert ist.

**9.** Pseudomonas-Immuntoxin nach Anspruch 5, worin mindestens 11 Aminosäuren in der Domäne Ia aus dem Exotoxin deletiert worden sind.

**10.** Pseudomonas-Immuntoxin nach Anspruch 5, worin mindestens 25 Aminosäuren in der Domäne Ia aus dem Exotoxin deletiert worden sind.

**11.** Pseudomonas-Immuntoxin nach Anspruch 5, worin mindestens 70 Aminosäuren in der Domäne Ia aus dem Exotoxin deletiert worden sind.

**12.** Pseudomonas-Immuntoxin nach Anspruch 5, worin mindestens 130 Aminosäuren aus der Domäne Ia aus dem Exotoxin deletiert worden sind.

**13.** Expressionsplasmid, umfassend DNA, welche eines der Proteine nach den Ansprüchen 1 bis 12 kodiert.

**14.** Verfahren zum Herstellen eines modifizierten Pseudomonas-Exotoxins nach Anspruch 1, umfassend das Transfizieren einer geeigneten Wirtszelle mit einem Plasmid, umfassend DNA, welche das modifizierte Exotoxin kodiert, und das Kultivieren der Wirtszelle unter Bedingungen, so daß das modifizierte Pseudomonas-Exotoxin produziert wird.

**15.** Verfahren zum Herstellen eines Immuntoxins nach Anspruch 5, umfassend
a) das Transfizieren einer geeigneten Wirtszelle mit einem Plasmid, welches das modifizierte Pseudomonas-Exotoxin nach Anspruch 1 kodiert
b) das Kultivieren der Wirtszelle unter Bedingungen, so daß das modifizierte Pseudomonas-Exotoxin produziert wird, und
c) das Konjugieren des modifizierten Exotoxins mit einem Antikörper, um ein Immuntoxin herzustellen.

**16.** Verfahren nach Anspruch 15, worin das modifizierte Pseudomonas-Exotoxingen die Domänen Ib, II und III des Pseudomonas-Exotoxins enthält.

**17.** Verfahren zum Herstellen eines Konjugats aus einem zielorientierten Träger und Pseudomonas-Exotoxin nach Anspruch 2, umfassend das Transfizieren einer geeigneten Wirtszelle mit einem Plasmid, umfassend eine DNA-Sequenz, welche das Konjugat mit dem zielorientierten Träger kodiert, und das Kultivieren der Wirtszelle unter Bedingungen, so daß ein Konjugat aus einem zielorientierten Träger und Pseudomonas-Exotoxin produziert wird.

**18.** Verfahren zum Herstellen einer Zusammensetzung, welche für die Verabreichung an ein Säugetier zum Erreichen einer zielorientierten zytotoxischen Aktivität in dem Säugetier geeignet ist, worin die Zusammensetzung ein Konjugat nach einem der Ansprüche 2 bis 12 in einem pharmazeutisch annehmbaren Träger umfaßt, umfassend das Mischen des Konjugats und des pharmazeutisch annehmbaren Trägers in einer an sich bekannten Weise.

**19.** Rekombinantes Plasmid, umfassend DNA, welche die Aminosäuren 253 bis 364 der Domäne II des Pseudomonas-Exotoxins kodiert.

**20.** Rekombinantes Plasmid nach Anspruch 19, worin das Plasmid pJH12 ist, welches unter der ATCC-Nr. 67205 hinterlegt ist.

**21.** Rekombinantes Plasmid, umfassend DNA, welche ein modifiziertes Pseudomonas-Exotoxin nach Anspruch 1 kodiert, fusioniert an DNA, welche mindestens die Aminosäuren 35 bis 223 von Interleukin 2 kodiert.

**22.** Rekombinantes Plasmid nach Anspruch 21, worin das Plasmid pHL-1 ist, welches unter der ATCC-Nr. 67206 hinterlegt ist.

**23.** Rekombinantes Plasmid, umfassend DNA, welche ein modifiziertes Pseudomonas-Exotoxin nach Anspruch 1 kodiert, fusioniert an DNA, welche $\alpha$-TGF kodiert.

**24.** Rekombinantes Plasmid nach Anspruch 23, worin das Plasmid pVC33 ist, welches unter der ATCC-Nr. 67207 hinterlegt ist.

**25.** Rekombinantes Plasmid, umfassend DNA, welche ein modifiziertes Pseudomonas-Exotoxin nach Anspruch 1 kodiert.

**26.** Rekombinantes Plasmid nach Anspruch 25, worin das Plasmid pJH8 ist, welches unter der ATCC-Nr. 67208 hinterlegt ist.

**27.** Rekombinantes Plasmid, umfassend DNA, welche die strukturelle Domäne Ia des Pseudomonas-Exotoxins kodiert.

**28.** Rekombinantes Plasmid nach Anspruch 27, worin das Plasmid pJH14 ist, welches unter der ATCC-Nr. 67209 hinterlegt ist.

**29.** Modifiziertes Pseudomonas-Exotoxin nach Anspruch 1, worin das Exotoxin genetisch modifiziert worden ist.

**30.** Konjugat aus einem Antigen und Toxin nach Anspruch 3, worin das Exotoxin durch rekombinante Techniken modifiziert worden ist.

**31.** Verwendung eines Exotoxins nach Anspruch 1 oder eines Exotoxins, welches durch eines der Plasmide nach den Ansprüchen 19 bis 28 kodiert ist, für die Herstellung eines Impfstoffes für aktive Immunisierung.

**32.** Verwendung eines Konjugats nach einem der Ansprüche 2 bis 12 für die Herstellung eines Medikaments zum Behandeln von Krebs.

**Revendications**
**Revendications pour les Etats contractants suivants : BE, CH, DE, FR, GB, IT, LI, LU, NL, SE**

1. Exotoxine de Pseudomonas modifiée qui comprend une activité de ribosilation de l'ADP et la capacité de se transloquer au travers d'une membrane cellulaire et dans laquelle l'exotoxine comprend une modification dans le domaine de fixation du récepteur Ia de la toxine native suffisante pour rendre la toxine modifiée moins toxique pour les cellules humaines ou animales in vitro et moins toxique pour le foie quand on l'administre in vivo, par rapport à une exotoxine de Pseudomonas non modifiée.

2. Conjugué toxine-véhicule permettant de cibler convenant à l'utilisation chez les hommes ou les mammifères comprenant une toxine fusionnée à un véhicule permettant de cibler dans lequel ladite toxine est une exotoxine de Pseudomonas modifiée selon la revendication 1.

3. Conjugué de la revendication 2, dans lequel ledit véhicule permettant de cibler est choisi dans le groupe constitué d'un antigène, d'un anticorps, d'un facteur de croissance, d'une hormone, d'une lymphokine, d'une protéine de reconnaissance cellulaire polypeptidique, d'une protéine sérique, d'une protéine sérique modifiée, d'une endorphine, d'une asialoglycoprotéine, de l'insuline, du glucagon, d'une hormone lutéinisante, d'un facteur de croissance des fibroblastes, d'un facteur de croissance des nerfs, d'une hormone de stimulation des mélanocytes, de la bombésine, et d'une lectine.

4. Conjugué de la revendication 2, dans lequel le véhicule permettant de cibler est choisi dans le groupe comprenant l'interleukine 2 et le facteur de croissance transformant alpha et une hormone peptidique.

5. Immunotoxine convenant à l'utilisation chez les humains ou les animaux comprenant une toxine fusionnée à un anticorps permettant de reconnaître les cellules malades ou causant des maladies, dans laquelle ladite toxine est une exotoxine de Pseudomonas modifiée selon la revendication 1.

6. Immunotoxine de la revendication 5, dans laquelle ladite exotoxine contient les domaines Ib, II et III de l'exotoxine de Pseudomonas.

7. Immunotoxine de la revendication 5, dans laquelle ladite exotoxine est l'exotoxine de Pseudomonas recombinante codée par le plasmide pJH8 déposé sous le numéro ATCC 67208.

8. Immunotoxine de Pseudomonas selon la revendication 5, dans laquelle au moins un acide aminé est supprimé du domaine Ia de ladite exotoxine.

9. Immunotoxine de Pseudomonas selon la revendication 5, dans laquelle au moins 11 acides aminés du domaine Ia sont supprimés de ladite exotoxine.

10. Immunotoxine de Pseudomonas selon la revendication 5, dans laquelle au moins 25 acides aminés du domaine Ia sont supprimés de ladite exotoxine.

11. Immunotoxine de Pseudomonas selon la revendication 5, dans laquelle au moins 70 acides aminés du domaine Ia sont supprimés de ladite exotoxine.

12. Immunotoxine de Pseudomonas selon la revendication 5, dans laquelle au moins 130 acides aminés du domaine Ia sont supprimés de ladite exotoxine.

13. Plasmide d'expression comprenant de l'ADN codant pour l'une quelconque des protéines des revendications 1 à 12.

14. Procédé de production d'une exotoxine de Pseudomonas modifiée selon la revendication 1 comprenant la transfection d'une cellule hôte convenable par un plasmide comprenant de l'ADN codant pour ladite exotoxine modifiée et la culture de ladite cellule hôte dans des conditions telles que l'exotoxine de Pseudomonas modifiée soit produite.

15. Procédé de production d'une immunotoxine selon la revendication 5 comprenant

23

EP 0 261 671 B1

a) la transfection d'une cellule hôte convenable par un plasmide codant pour l'exotoxine de Pseudomonas modifiée, selon la revendication 1.
b) la culture de ladite cellule hôte dans des conditions telles que l'exotoxine de Pseudomonas modifiée soit produite, et
c) la conjugaison de ladite exotoxine avec un anticorps pour produire une immunotoxine.

16. Procédé de la revendication 15, dans lequel le gène de ladite exotoxine de Pseudomonas modifiée contient les domaines Ib, II et III de l'exotoxine de Pseudomonas.

17. Procédé de production d'un conjugué véhicule permettant de cibler-exotoxine de Pseudomonas modifiée selon la revendication 2, comprenant la transfection d'une cellule hôte convenable par un plasmide comprenant une séquence d'ADN codant pour ledit conjugué de véhicule permettant de cibler et la culture de ladite cellule hôte dans des conditions telles que le conjugué véhicule permettant de cibler-exotoxine de Pseudomonas soit produite.

18. Composition convenant à l'administration à un mammifère destinée à obtenir une activité cytotoxique ciblée dans ledit mammifère, dans laquelle la composition comprend un conjugué selon l'une quelconque des revendications 2 à 12 dans un véhicule acceptable du point de vue pharmaceutique.

19. Plasmide recombinant comprenant de l'ADN codant pour les acides aminés 253 à 364 du domaine II de l'exotoxine de Pseudomonas.

20. Plasmide recombinant de la revendication 19, dans lequel le plasmide est pJH12 déposé sous le numéro ATCC 67205.

21. Plasmide recombinant comprenant de l'ADN codant pour une exotoxine de Pseudomonas modifiée selon la revendication 1 fusionné à de l'ADN codant au moins pour les acides aminés 35 à 223 de l'interleukine 2.

22. Plasmide recombinant de la revendication 21, dans lequel le plasmide est pHL-1 déposé sous le numéro ATCC 67206.

23. Plasmide recombinant comprenant de l'ADN codant pour une exotoxine de Pseudomonas modifiée selon la revendication 1 fusionné à de l'ADN codant pour l'alpha TGF.

24. Plasmide recombinant de la revendication 23, dans lequel le plasmide est pVC33 déposé sous le numéro ATCC 67207.

25. Plasmide recombinant comprenant de l'ADN codant pour une exotoxine de Pseudomonas modifiée selon la revendication 1.

26. Plasmide recombinant de la revendication 25, dans lequel le plasmide est pJH8 déposé sous le numéro ATCC 67208.

27. Plasmide recombinant comprenant de l'ADN codant pour le domaine structurel Ia de l'exotoxine de Pseudomonas.

28. Plasmide recombinant de la revendication 27, dans lequel le plasmide est pJH14 déposé sous le numéro ATCC 67209.

29. Exotoxine de Pseudomonas modifiée selon la revendication 1 dans laquelle ladite exotoxine a été génétiquement modifiée.

30. Conjugué antigène-toxine selon la revendication 3 dans lequel ladite exotoxine a été modifiée par des techniques de recombinaison.

31. Utilisation d'une exotoxine selon la revendication 1 ou d'une exotoxine codée par l'un quelconque des plasmides des revendications 19 à 28 pour la préparation d'un vaccin pour immunisation active.

24

**32.** Utilisation d'un conjugué selon l'une quelconque des revendications 2 à 12 pour la préparation d'un médicament de traitement du cancer.

**Revendications pour les Etats contractants suivants : ES, AT**

**1.** Procédé de production d'une exotoxine de Pseudomonas modifiée qui comprend une activité de ribosilation de l'ADP et la capacité de se transloquer au travers d'une membrane cellulaire et dans laquelle l'exotoxine comprend une modification dans le domaine de fixation du récepteur Ia de la toxine native suffisante pour rendre la toxine modifiée moins toxique pour les cellules humaines ou animales in vitro et moins toxique pour le foie quand on l'administre in vivo, par rapport à une exotoxine de Pseudomonas non modifiée, comprenant la transfection d'une cellule hôte convenable par un plasmide comprenant de l'ADN codant pour ladite exotoxine modifiée et la culture de ladite cellule hôte dans des conditions telles que l'exotoxine de Pseudomonas modifiée soit produite.

**2.** Procédé de production d'un conjugué toxine-véhicule permettant de cibler convenant à l'utilisation chez les hommes ou les mammifères, comprenant une toxine fusionnée à un véhicule permettant de cibler dans lequel ladite toxine est une exotoxine de Pseudomonas modifiée selon la revendication 1, comprenant la transfection d'une cellule hôte convenable par un plasmide comprenant de l'ADN codant pour ladite exotoxine modifiée et la culture de ladite cellule hôte dans des conditions telles que l'exotoxine de Pseudomonas modifiée soit produite.

**3.** Procédé de la revendication 2, dans lequel ledit véhicule permettant de cibler est choisi dans le groupe constitué d'un antigène, d'un anticorps, d'un facteur de croissance, d'une hormone, d'une lymphokine, d'une protéine de reconnaissance cellulaire polypeptidique, d'une protéine sérique, d'une protéine sérique modifiée, d'une endorphine, d'une asialoglycoprotéine, de l'insuline, du glucagon, d'une hormone lutéinisante, d'un facteur de croissance des fibroblastes, d'un facteur de croissance des nerfs, d'une hormone de stimulation des mélanocytes, de la bombésine, et d'une lectine.

**4.** Procédé de la revendication 2, dans lequel le véhicule permettant de cibler est choisi dans le groupe comprenant l'interleukine 2 et le facteur de croissance transformant alpha et une hormone peptidique.

**5.** Procédé de production d'une immunotoxine convenant à l'utilisation chez les humains ou les animaux comprenant une toxine fusionnée à un anticorps capable de reconnaître les cellules malades ou causant des maladies, dans lequel ladite toxine est une exotoxine de Pseudomonas modifiée selon la revendication 1, comprenant
a) la transfection d'une cellule hôte convenable par un plasmide codant pour l'exotoxine de Pseudomonas modifiée, selon la revendication 1.
b) la culture de ladite cellule hôte dans des conditions telles que l'exotoxine de Pseudomonas modifiée soit produite, et
c) la conjugaison de ladite exotoxine avec un anticorps pour produire une immunotoxine.

**6.** Procédé de la revendication 5, dans lequel ledit gène d'exotoxine de Pseudomonas modifiée contient les domaines Ib, II et III de l'exotoxine de Pseudomonas.

**7.** Procédé de la revendication 5, dans lequel ledit plasmide est pJH8. déposé sous le numéro ATCC 67208.

**8.** Procédé de la revendication 5, dans lequel au moins un acide aminé est supprimé du domaine Ia de ladite exotoxine.

**9.** Procédé de la revendication 5, dans lequel au moins 11 acides aminés du domaine Ia sont supprimés de ladite exotoxine.

**10.** Procédé de la revendication 5, dans lequel au moins 25 acides aminés du domaine Ia sont supprimés de ladite exotoxine.

**11.** Procédé de la revendication 5, dans lequel au moins 70 acides aminés du domaine Ia sont supprimés de ladite exotoxine.

**12.** Procédé de la revendication 5, dans lequel au moins 130 acides aminés du domaine Ia sont supprimés de ladite exotoxine.

**13.** Procédé de préparation d'un plasmide d'expression comprenant de l'ADN codant pour l'une quelconque des protéines des revendications 1 à 12, comprenant la construction dudit plasmide d'expression d'une manière connue en soi.

**14.** Procédé de préparation d'une composition convenant à l'administration à un mammifère afin d'obtenir une activité cytotoxique ciblée dans ledit mammifère, dans lequel la composition comprend un conjugué selon l'une quelconque des revendications 2 à 12 dans un véhicule acceptable du point de vue pharmaceutique, comprenant le mélange dudit conjugué et ledit véhicule acceptable du point de vue pharmaceutique d'une manière connue en soi.

**15.** Procédé de préparation d'un plasmide recombinant comprenant de l'ADN codant pour les acides minés 253 à 364 du domaine II de l'exotoxine de Pseudomonas, comprenant la construction dudit plasmide recombinant d'une manière connue en soi.

**16.** Procédé de la revendication 15, dans lequel le plasmide est pJH12 déposé sous le numéro ATCC 67205.

**17.** Procédé de préparation d'un plasmide recombinant comprenant de l'ADN codant pour une exotoxine de Pseudomonas modifiée selon la revendication 1 fusionné à de l'ADN codant au moins pour les acides aminés 35 à 223 de l'interleukine 2, comprenant la construction dudit plasmide recombinant d'une manière connue en soi.

**18.** Procédé de la revendication 17, dans lequel le plasmide est pHL-1 déposé sous le numéro ATCC 67206.

**19.** Procédé de préparation d'un plasmide recombinant comprenant de l'ADN codant pour une exotoxine de Pseudomonas modifiée selon la revendication 1 fusionné à de l'ADN codant pour l'alpha TGF, comprenant la construction dudit plasmide recombinant d'une manière connue en soi.

**20.** Procédé de la revendication 19, dans lequel le plasmide est pVC33 déposé sous le numéro ATCC 67207.

**21.** Procédé de préparation d'un plasmide recombinant comprenant de l'ADN codant pour une exotoxine de Pseudomonas modifiée selon la revendication 1, comprenant la construction dudit plasmide recombinant d'une manière connue en soi.

**22.** Procédé de la revendication 21, dans lequel le plasmide est pJH8 déposé sous le numéro ATCC 67208.

**23.** Procédé de préparation d'un plasmide recombinant comprenant de l'ADN codant pour le domaine structurel Ia de l'exotoxine de Pseudomonas, comprenant la construction dudit plasmide recombinant d'une manière connue en soi.

**24.** Procédé de la revendication 23, dans lequel le plasmide est pJH14 déposé sous le numéro ATCC 67209.

**25.** Procédé de préparation d'une exotoxine de Pseudomonas modifiée selon la revendication 1, comprenant la modification génétique de ladite exotoxine.

**26.** Procédé de préparation d'un conjugué antigène-toxine selon la revendication 3, comprenant la modification de ladite exotoxine par des techniques de recombinaison.

**27.** Procédé de préparation d'un vaccin pour l'immunisation active qui comprend une exotoxine obtenue selon la revendication 1 ou une exotoxine codée par l'un quelconque des plasmides selon les revendication 15 à 24, comprenant la formulation dudit vaccin d'une manière connue en soi.

**28.** Procédé de préparation d'un médicament pour le traitement du cancer qui comprend un conjugué obtenu selon l'une quelconque des revendications 2 à 12, comprenant la formulation dudit médicament d' une manière connue en soi.

**Revendications pour l'Etat contractant suivant : GR**

**1.** Exotoxine de Pseudomonas modifiée qui comprend une activité de ribosilation de l'ADP et la capacité de se transloquer au travers d'une membrane cellulaire et dans laquelle l'exotoxine comprend une modification dans le domaine de fixation du récepteur Ia de la toxine native suffisante pour rendre la toxine modifiée moins toxique pour les cellules humaines ou animales in vitro et moins toxique pour le foie quand on l'administre in vivo, par rapport à une exotoxine de Pseudomonas non modifiée.

**2.** Conjugué toxine-véhicule permettant de cibler convenant à l'utilisation chez les hommes et les mammifères comprenant une toxine fusionnée à un véhicule permettant de cibler dans lequel ladite toxine est une exotoxine de Pseudomonas modifiée selon la revendication 1.

**3.** Conjugué de la revendication 2, dans lequel ledit véhicule permettant de cibler est choisi dans le groupe constitué d'un antigène, d'un anticorps, d'un facteur de croissance, d'une hormone, d'une lymphokine, d'une protéine de reconnaissance cellulaire polypeptidique, d'une protéine sérique, d'une protéine sérique modifiée, d'une endorphine, d'une asialoglycoprotéine, de l'insuline, du glucagon, d'une hormone lutéinisante, d'un facteur de croissance des fibroblastes, d'un facteur de croissance des nerfs, d'une hormone de stimulation des mélanocytes, de la bombésine, et d'une lectine.

**4.** Conjugué de la revendication 2, dans lequel le véhicule permettant de cibler est choisi dans le groupe comprenant l'interleukine 2 et le facteur de croissance transformant alpha et une hormone peptidique.

**5.** Immunotoxine convenant a l'utilisation chez les humains ou les animaux comprenant une toxine fusionnée à un anticorps permettant de reconnaître les cellules malades ou causant des maladies, dans laquelle ladite toxine est une exotoxine de Pseudomonas modifiée selon la revendication 1.

**6.** Immunotoxine de la revendication 5, dans laquelle ladite exotoxine contient les domaines Ib, II et III de l'exotoxine de Pseudomonas.

**7.** Immunotoxine de la revendication 5, dans laquelle ladite toxine est l'exotoxine de Pseudomonas recombinante codée par le plasmide pJH8 déposé sous le numéro ATCC 67208.

**8.** Immunotoxine de Pseudomonas selon la revendication 5, dans laquelle au moins un acide aminé est supprimé du domaine Ia de ladite exotoxine.

**9.** Immunotoxine de Pseudomonas selon la revendication 5, dans laquelle au moins 11 acides aminés du domaine Ia sont supprimés de ladite exotoxine.

**10.** Immunotoxine de Pseudomonas selon la revendication 5, dans laquelle au moins 25 acides aminés du domaine Ia sont supprimés de ladite exotoxine.

**11.** Immunotoxine de Pseudomonas selon la revendication 5, dans laquelle au moins 70 acides aminés du domaine Ia sont supprimés de ladite exotoxine.

**12.** Immunotoxine de Pseudomonas selon la revendication 5, dans laquelle au moins 130 acides aminés du domaine Ia sont supprimés de ladite exotoxine.

**13.** Plasmide d'expression comprenant de l'ADN codant pour l'une quelconque des protéines des revendications 1 à 12.

**14.** Procédé de production d'une exotoxine de Pseudomonas modifiée selon la revendication 1 comprenant la transfection d'une cellule hôte convenable par un plasmide comprenant de l'ADN codant pour ladite exotoxine modifiée et la culture de ladite cellule hôte dans des conditions telles que l'exotoxine de Pseudomonas modifiée soit produite.

**15.** Procédé de production d'une immunotoxine selon la revendication 5 comprenant

a) la transfection d'une cellule hôte convenable par un plasmide codant pour l'exotoxine de Pseudomonas modifiée, selon la revendication 1.

b) la culture de ladite cellule hôte dans des conditions telles que l'exotoxine de Pseudomonas modifiée soit produite, et

c) la conjugaison de ladite exotoxine avec un anticorps pour produire une immunotoxine.

**16.** Procédé de la revendication 15, dans lequel le gène de ladite exotoxine de Pseudomonas modifiée contient les domaines Ib, II et III de l'exotoxine de Pseudomonas.

**17.** Procédé de production d'un conjugué véhicule permettant de cibler-exotoxine de Pseudomonas modifiée selon la revendication 2 comprenant la transfection d'une cellule hôte convenable par un plasmide comprenant une séquence d'ADN codant pour ledit conjugué de véhicule permettant de cibler et la culture de ladite cellule hôte dans des conditions telles que le conjugué véhicule permettant de cibler-exotoxine de Pseudomonas soit produite.

**18.** Procédé de préparation d'une composition convenant à l'administration à un mammifère pour obtenir une activité cytotoxique ciblée dans ledit mammifère, dans laquelle la composition comprend un conjugué selon l'une quelconque des revendications 2 à 12 dans un véhicule acceptable du point de vue pharmaceutique. comprenant le mélange dudit conjugué et dudit véhicule acceptable du point de vue pharmaceutique d'une manière connue en soi.

**19.** Plasmide recombinant comprenant de l'ADN codant pour les acides aminés 253 à 364 du domaine II de l'exotoxine de Pseudomonas.

**20.** Plasmide recombinant de la revendication 19, dans lequel le plasmide est pJH12 déposé sous le numéro ATCC 67205.

**21.** Plasmide recombinant comprenant de l'ADN codant pour une exotoxine de Pseudomonas modifiée selon la revendication 1 fusionné à de l'ADN codant au moins pour les acides aminés 35 à 223 de l'interleukine 2.

**22.** Plasmide recombinant de la revendication 21, dans lequel le plasmide est pHL-1 déposé sous le numéro ATCC 67206.

**23.** Plasmide recombinant comprenant de l'ADN codant pour une exotoxine de Pseudomonas modifiée selon la revendication 1 fusionné à de l'ADN codant pour l'alpha TGF.

**24.** Plasmide recombinant de la revendication 23, dans lequel le plasmide est pVC33 déposé sous le numéro ATCC 67207.

**25.** Plasmide recombinant comprenant de l'ADN codant pour une exotoxine de Pseudomonas modifiée selon la revendication 1.

**26.** Plasmide recombinant de la revendication 25, dans lequel le plasmide est pJH8 déposé sous le numéro ATCC 67208.

**27.** Plasmide recombinant comprenant de l'ADN codant pour le domaine structurel Ia de l'exotoxine de Pseudomonas.

**28.** Plasmide recombinant de la revendication 27, dans lequel le plasmide est pJH14 déposé sous le numéro ATCC 67209.

**29.** Exotoxine de Pseudomonas modifiée selon la revendication 1 dans laquelle ladite exotoxine a été génétiquement modifiée.

**30.** Conjugué antigène-toxine selon la revendication 3 dans lequel ladite exotoxine a été modifiée par des techniques de recombinaison.

31. Utilisation d'une exotoxine selon la revendication 1 ou d'une exotoxine codée par l'un quelconque des plasmides des revendications 19 à 28 pour la préparation d'un vaccin pour immunisation active.

32. Utilisation d'un conjugué selon l'une quelconque des revendications 2 à 12 pour la préparation d'un médicament de traitement du cancer.

FIG.1

FIG. 2

FIG.1

PE / PE$_{45}$ - HB 21  Vs KB 3-1

● PE - HB 21 (TWO EXPERIMENTS)
△ PE$_{45}$ - HB 21

CONTROL PROTEIN SYNTHESIS %

ng/ml

FIG. 3B

SWISS 3T3

- ● —● PE −HB 21
- ○ —○ PE₄₅ −HB 21
- ▲ —▲ PE
- △ —△ PE₄₅

CONTROL PROTEIN SYNTHESIS %

0,52ng/ml

ng/ml

EP 0 261 671 B1